Europäisches Patentamt

European Patent Office

Office européen des brevets

(11) Veröffentlichungsnummer: **0 273 333**
A2

(19)

# (12) EUROPÄISCHE PATENTANMELDUNG

(21) Anmeldenummer: 87118904.9

(22) Anmeldetag: 19.12.87

(51) Int. Cl.⁴: **G01N 33/569** , C12N 5/00 , C12P 21/00 , G01N 33/546 , G01N 33/577 , //C12N15/00

(30) Priorität: 02.01.87 DE 3700049

(43) Veröffentlichungstag der Anmeldung:
06.07.88 Patentblatt 88/27

(84) Benannte Vertragsstaaten:
AT BE CH DE ES FR GB GR IT LI LU NL SE

(71) Anmelder: **Biotest AG**
**Flughafenstrasse 4**
**D-6000 Frankfurt am Main 73(DE)**

(72) Erfinder: **Mauch, Harald, Dr. med.**
**Lückowstrasse 10a**
**D-1000 Berlin 38(DE)**
Erfinder: **Wagner, Sonja, Dr. Dipl.-Biochem.**
**Waltroper Platz 5**
**D-1000 Berlin 45(DE)**
Erfinder: **Sonneborn, Hans, H., Dr. phil. nat.**
**Im Birkeneck 70**
**D-6056 Heusenstamm(DE)**
Erfinder: **Horn, Jürgen, Dr. rer. nat.**
**Kurt-Schumacher-Ring 83**
**D-6073 Egelsbach-Bayerseich(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**D-6230 Frankfurt am Main 80(DE)**

(54) **Verfahren zum immunologischen Nachweis von Mykobakterien im Sputum sowie monoklonale Antikörper zur Durchführung des Verfahrens.**

(57) Mykobakterien lassen sich direkt im Sputum immunologisch nachweisen, wenn man eine verflüssigte Sputumprobe einer 2 - 4 minütigen Wärme-Tensidbehandlung unterwirft, die so behandelte Sputumprobe ausstreicht, den Ausstrich mit einem markierten Polyklonalen oder monoklonalen Antikörper gegen Mykobakterien in Gegenwart von Feuchtigkeit inkubiert und die Probe einer für die Markierung geeigneten immunologischen Nachweismethode unterwirft.
Besonders geeignete monoklonale Antikörper stammen von Hybridoma-Zell-Linien BS 100, 102, 104 und 107.

EP 0 273 333 A2

## Verfahren zum immunologischen Nachweis von Mykobakterien im Sputum sowie monoklonale Antikörper zur Durchführung des Verfahrens

Die Erfindung betrifft ein Verfahren zum immunologischen Nachweis von Mykobakterien im Sputum sowie monoklonale Antikörper zur Durchführung des Verfahrens.

Immunologische Schnelltests von mykobakteriellen Antigenen wurden bisher nur mit Liquor oder Gewebsschnitten als Probematerial beschrieben (The Lancet, 17.September 1983, S.651-652; J. of Clinical Microbiology Vol.20, No.3, September 1984, S.533-535; Am.J.Vet.Res., Vol.42, No.10, Oktober 1981, S.1814-1815). In der EP-A 0 174 805 wird der Nachweis von Mykobakterium tuberculosis-Antigen beschrieben, welches als Immunkomplex im Serum vorkommt. Antigen direkt aus Sputum, Pleuraflüssigkeit und sonstigen Körperproben wird nicht bestimmt. Die Aufarbeitung des Serums beinhaltet die Dissoziation der Immunkomplexe, Denaturierung von Serummaterial mit anschließender Dialyse des Überstandes. Nur derartiges Material kann im Test eingesetzt werden. Auch bei Untersuchungen von Mykobakterien aus Kultur muß erst ein Extrakt aus den Mykobakterien angefertigt werden, bevor das Material im immunologischen Test eingesetzt werden kann.

Die meisten aller zu untersuchenden Proben bestehen jedoch aus Sputum. Im Sputum lassen sich frühzeitig im Krankheitsverlauf die infektiösen intakten Mykobakterien nachweisen. Im Gegensatz dazu entstehen Immunkomplexe im Serum, wie sie in der EP-A 0 174 805 verwendet werden, erst im späteren Verlauf der Krankheit, wenn durch Immunantwort des Organismus Mykobakterien zerfallen und die nachgewiesenen Bruchstücke als Immunkomplexe im Serum auftauchen.

Der Nachweis von Mykobakterien im Sputum ist deshalb die bevorzugte Methode.

Der Nachweis einer Mykobakterieninfektion im Sputum beruht bisher auf der Anfärbung von Mykobakterien im Sputum nach der Methode von Ziehl-Neelson. Da diese Methode nicht empfindlich genug ist, erfolgt nach Dekontamination und Verflüssigung von Sputum eine Kultur von Mykobakterien auf geeigneten Nährböden (Lehrbuch der medizinischen Mikrobilogie von H.J.Otte und H.Brandis S.356-358).

Bisher gibt es keine Methode, um direkt im Sputum eine immunologische Reaktion durchzuführen.

Der Erfindung liegt die Aufgabe zugrunde, ein Verfahren bereitzustellen,mit dessen Hilfe eine Mykobakterieninfektion direkt im Sputum auf einfache, sichere Weise immunologisch nachgewiesen werden kann.

Diese Aufgabe wird erfindungsgemäß durch die kennzeichnenden Merkmale des Anspruchs 1 gelöst.

Es war überraschend, daß ein immunologischer Nachweis einer auf übliche Weise verflüssigten Sputumprobe sich dann erfolgreich durchführen läßt, wenn man nach Zentrifugieren der verflüssigten Probe das Sediment einer Wärmebehandlung in Gegenwart einer gewissen Auswahl an Tensiden in einer bestimmten vorgegebenen Zeit unterwirft. Die vorgegebene Behandlungszeit muß dabei genau eingehalten werden. Sie muss einerseits ausreichen, um eine grösstmögliche Denaturierung zu bewirken, andererseits darf sie jedoch nicht so lange dauern, dass die Mykobakterien absterben. Eine Behandlungszeit von 2 - 4 Minuten bei 80 - 100°C erwies sich als geeignet. Bei höheren Temperaturen wird man mit kürzeren Behandlungszeiten innerhalb der beanspruchten Temperatur-und Zeitbereiche auskommen und umgekehrt. Als besonders brauchbare Kombination von Behandlungszeit und Temperatur erwiesen sich 2 1/2 Minuten bei 100°C.

Mit Hilfe des erfindungsgemäßen Verfahrens lassen sich Mykobakterien aus Kultur direkt im immunologischen Test einsetzen.

Es lassen sich ferner direkt klinische Sputumproben einsetzen, wobei die darin enthaltenen Mykobakterien nach Verflüssigung und Wärme-Tensid-Behandlung des Sputums direkt immunologisch nachgewiesen werden können.

Es müssen keine Extrakte aus diesen Mykobakterien angefertigt werden, da die verwendeten Antikörper direkt mit Antigen auf der Oberfläche der intakten Mykobakterien reagieren. Zur Feststellung von M.tuberculosis werden Antikörper verwendet, welche ausschließlich mit M.tuberculosis reagieren. Zur Feststellung von ubiquitär vorkommenden ("atypischen") Mykobakterien werden Antikörper verwendet, welche allgemein mit Mykobakterien reagieren, jedoch nicht mit anderen Bakterien.

Nach Austestung der klinischen Proben mit den mykobakterien-spezifischen Antikörpern, ergeben sich also alle Mykobakterien. Nach weiterer Austestung mit M.tuberculosis-spezifischen Antikörpern wird M.tuberculosis separat diagnostiziert und die weiteren positiven Fälle der vorhandenen Gruppe sind die Mykobakteriosen mit ubiquitär vorkommenden ("atypischen") Mykobakterien.

Selbstverständlich kann auch ausschließlich auf M.tuberculosis geprüft werden, wenn die sonstigen Mykobakterien nicht interessieren.

Ein weiterer Vorteil ist, daß bereits frühzeitig im Krankheitsverlauf die infektiösen intakten Mykobakterien nachgewiesen werden können.

Beim erfindungsgemäßen Verfahren können sowohl polyklonale als auch monoklonale Antikörper eingesetzt werden.

Besonders bevorzugt wird die Verwendung von monoklonalen Antikörpern, welche ausschließlich mit der Oberfläche von Mykobakterien reagieren. Es werden zum einen Antikörper eingesetzt, welche allgemein mit Mykobakterien reagieren (BS 107 ) und Antikörper, welche spezifisch mit M.tuberculosis reagieren (BS 100, 102 und 104).

Nach einer weiteren Ausführungsform lassen sich an Latexpartikel gebundene monoklonale Antikörper verwenden. Diese Latexpartikel können dann mit dem verflüssigten, Wärme-Tensid behandelten Sputummaterial inkubiert werden. Falls zum Zwecke des immunologischen Nachweises mit Peroxidase markiert wird, ist eventuell vorhandene Gewebsperoxidase zu inaktivieren. Danach folgt ein Waschschritt. Der Waschschritt kann z.B. durch Absaugen und Nachspülen mit Puffer in Mikrofiltrationsplatten oder -streifen erfolgen. Danach erfolgt eine Inkubation mit spezifischen monoklonalen Antikörpern gegen M.tuberculosis. Nach einem weiteren Waschschritt erfolgt eine für die jeweilige Markierung geeignete immunologische Nachweismethode.

In einer weiteren Ausführungsform der Erfindung werden monoklonale Antikörper gegen verschiedene Epitope auf der Oberfläche der Mykobakterien verwendet und zur Bindung an die Latexpartikel Antikörper gegen andere Epitope ausgewählt als die Epitope, mit denen die Antikörper reagieren, welche markiert werden. Dadurch kann der Inkubationsschritt mit dem verflüssigten, Wärme-Tensid behandelten Sputummaterial gleichzeitig mit der Inkubation mit markiertem Antikörper erfolgen.

Die Vorbehandlung, d.h. Sputumverflüssigung und Dekontaminierung ist allgemein bekannt und wird u.a. im obengenannten Lehrbuch der medizinischen Mikrobiologie und in DIN 58943, Teil 3 beschrieben. Gebräuchliche Vorbehandlungsmittel sind u.a. Na-Diisobutylnaphthalinsulfonat (Nekal-BX), N-Acetyl-L-cystein, Natriumlaurylsulfat, Pankreatin/Desogen (Desogen = Methylphenyldodecyl-trimethylammoniumethosulfat) und Sputufluol (NaOH + Natriumhypochlorit).

Als Tenside zur Wärme-Tensidbehandlung eignen sich neben Natriumdodecylsulfat zwitterionische Sulfobetaine, wie sie in der Firmenzeitschrift CALBIOCHEM Biochemicals, Behring Diagnostics Division of American Hoechst, Brand Detergents, S. 308 ff (1985) beschrieben werden, wie z.B. N-Octyl-, N-Decyl-, N-Dodecyl-, N-Tetradecyl-oder N-Hexadecyl-N,N-dimethyl-3-ammonio-1-propansulfonat.

Zu den geeigneten nichtionischen Tensiden gehören Polyglykolether, wie sie beispielsweise in der Alphabetic List of All Compounds der Firma Sigma, S.1095 ( 1986 ) beschrieben werden, die Polyoxyethylenether, zu denen die Lauryl-, Cetyl-, Oleyl-, Stearyl-und Tridecylether gehören, sowie die Polyoxyethylensorbitane, wie sie in der gleichen Druckschrift auf S.1006 sowie letztere auch im Merck-Katalog ( 1984 ) S.288 und im Roth-Katalog ( 1985 ), S.345 beschrieben werden, wozu die Mono-, Di-und Trilaurate, -palmitate, -stearate und -oleate gehören. Weitere geeignete nichtionische Tenside sind die Glucopyranoside, wie z.B. Octyl-$\beta$-D-glucopyranosid.

Die Markierung der Antikörper gegen Mykobakterum tuberculosis bzw. allgemein gegen Mykobakterien kann nach den bekannten Methoden radioaktiv (z.B. mit J 125), mit Luminiszenzmarkierung oder mit Enzymen (M.Gellerich, "Enzyme Immunoassay in Clinical Chemistry", J.Clin.Chem.Clin.Biochem., Vol.18 (1980), S.200) oder mit Fluoreszenzfarbstoffen, wie Fluoresceinisothiocyanat (FITC), Sulfonylchlorid von Dimethylaminonaphthalin-5-sulphonsäure (DANS) oder Tetramethylrhodaminisothiocyanat (TMRITC) (Cellular Pathology Technique, 4.Ed. ( 1985, S.349-365) erfolgen. Bevorzugte Enzyme sind z.B. Peroxidase und alkalische Phosphatase.

Als Nachweismethode für radioaktive Markierung können Röntgenfilme oder Gammazähler verwendet werden.

Bei Luminiszenzmarkierung werden Luminometer verwendet.

Die Enzymmarkierung kann mit geeigneten Farbsubstraten wie DAB (Diaminobenzidine), Aminoethylcarbazol, Tetramethylbenzidin, PDP (Phenylendiaminpyrocatechol) oder weitere dem Fachmann bekannten sichtbar gemacht werden und danach im Lichtmikroskop ausgewertet werden (Cellular Pathology Technique, S.369-370).

Fluoreszenzmarkierung wird in einem Fluoreszenzmikroskop mit einem geeigneten Filter ausgewertet.

Eine Signalverstärkung läßt sich durch die Anwendung der Streptavidin bzw. Avidin-Biotion Verbrauchstechnik erreichen. Diese kann für Enzymmarkierung, Fluoreszenzmarkierung und auch Luminiszenz-oder radioaktive Markierung verwendet werden. Details dieser Technik sind in Immunolabelling for Electron Microscopy, Amsterdam (1984), S.95-111 und J.of Histochemistry and Cytochemistry 27 (1979), Nr.8, S.1131-1139 beschrieben.

Nachstehende Beispiele dienen der weiteren Erläuterung der Erfindung.

Rezept 1: SDS-1x-Puffer5 ml 0,2M Phosphatpuffer pH 7,2
1 ml 2-Mercapto-Ethanol
1 g Natriumdodecylsulfat (SDS)

auf 100 ml mit Aqua dest. auffüllen.

Rezept 2: PBS-Polyoxyethylensorbitanmonolaurat-Puffer0,15 M Phosphat-gepufferte Salzlösung (PBS)
8 g Natriumchlorid
0,2 g Kaliumchlorid
1,424 g Di-natriumhydrogenphosphat x $H_2O$
0,2 g Kaliumdihydrogenphosphat

alles in einem Liter Aqua dest. lösen und 0,5 ml Polyoxyethylensorbitanmonolaurat zusetzen.

Rezept 3: Enzym-Substrat-Färbelösung

Vor Gebrauch frisch ansetzen!

40mg 3-Amino-9-ethyl-carbazol
in 5 ml Dimethylformamid lösung und in 100 ml 0,01 M Tris-HCl-Puffer, pH 8,0 verrühren und 50 µl $H_2O_2$
zusetzen.

Rezept 4: Sputumverflüssigung

1 Teil einer Sputumprobe wird mit 2 bis 3 Teilen Na-Diisobutylnaphthalinsulfonsäure versetzt und kräftig geschüttelt. Danach wird die Probe bei 37,5°C 1 Std. inkubiert und nochmals gut durchgemischt. Anschließend wird die Probe nochmals 30 Minuten bei 80°C inkubiert, wodurch vollständige Verflüssigung und ·Keimabtötung (Dekontaminierung) stattfindet. Die verflüssigte Probe wird mit Hilfe einer Laborzentrifuge bei 3 500 U/min. über 20 Min. zentrifugiert, der Überstand abgegossen und das Sediment gesammelt.

Beispiel 1

Immunanfärbung von Mykobakterien im Sputum mit polyklonalen Antiseren

A. Vorbereitung des Sputums:

1 Teil des gemäß Rezept 4 hergestellten Sediments der Sputumprobe wird mit 2-3 Teilen des SDS-1x-Puffers gemäß Rezept 1 versetzt und 2,5 Min. im Wasserbad auf 100°C erhitzt. Gegebenenfalls vorhandene grobe Schleimflocken in der Probe werden durch kurze Zentrifugation (etwa 5 Min.) bei etwa 1200 U/min. abgetrennt. Der Überstand wird zur weiteren diagnostischen Untersuchung eingesetzt.

B. Herstellen der Präparate:

10 µl der gemäß A. behandelten verflüssigten Sputumprobe werden auf dem Objektträger ausgestrichen und an der Luft getrocknet. Alternativ kann der Ausstrich auch getrocknet werden, indem man ihn dreimal durch die Flamme eines Bunsenbrenners zieht. Anschließend wird der Ausstrich 10 min in 4°C kaltem Aceton fixiert und während 1 min in PBS-Polyoxyethylensorbitanmonolaurat-Puffer gemäß Rezept 2 rehydriert. Da der Detektionsantikörper mit Peroxidase (POD) markiert vorliegt, wird eventuell vorhandene Gewebsperoxidase vor der Färbung inaktiviert. Dazu wird der Objektträger mit 4%igem $H_2O_2$ vollständig bedeckt 5 min lang in einer feuchten Kammer inkubiert. Danach werden die Proben in einem PBS-Polyoxyethylensorbitanmonolaurat-Pufferbad dreimal gewaschen und anschließend sorgfältig mit Fließpapier getrocknet. Die Lage der Probe wird mit einem Diamantschreiber auf dem Objektträger durch einen Kreis markiert. Dies hat drei Vorteile: 1) nach dem Rehydrieren sind die Ausstriche oft schlecht erkennbar, 2) die Probe wird beim Trocknen des Objektträgers nicht so leicht versehentlich weggewischt und 3) der Antikörper wird bei der Färbung besser in der richtigen Position aufgetragen.

## C. Immunanfärbung der Präparate:

Von hier an müssen die Präparate bis zum Ende feucht gehalten werden. Die Inkubation des 1. Antikörpers erfolgt 20 min bei Raumtemperatur in einer Probe mit polyklonalem Meerschweinchenserum, selbst hergestellt nach Immunisierung mit Mykobakterium tuberculosis, Referenzstamm $H_{37}Rv$, 1 h 80°C inaktiviert, 1:80 verdünnt in PBS-Polyoxyethylensorbitanmonolaurat-Puffer und in einer anderen Probe mit polyklonalem Kaninchen anti-BCG-Antikörper (Fa.Dako B-124) 1:200 verdünnt in PBS-Polyoxyethylensorbitanmonolaurat-Puffer. Der Ausstrich wird vollständig mit 200 µl Antikörperverdünnung pro Objektträger bedeckt. Zum Entfernen von überschüssigen Antikörpern wird dreimal mit PBS-Polyoxyethylensorbitanmonolaurat-Puffer gewaschen.

Zum Nachweis des gebundenen 1. Antikörpers wird der Ausstrich mit 200 µl Peroxidase-markiertem Protein A aus Staph. aureus (Fa.Medac, HP-02), 1:700 verdünnt in PBS-Polyoxyethylensorbitanmonolaurat-Puffer bedeckt und weitere 20 min in einer feuchten Kammer inkubiert. Zum Entfernen von überschüssigem Protein A wird das Präparat dreimal mit PBS-Polyoxyethylensorbitanmonolaurat-Puffer gewaschen.

Die Entwicklung der Mykobakterienfärbung erfolgt durch Schaukeln der Objektträger in der Enzym-Substrat-Färbelösung gemäß Rezept 3 während 5-10 min. Die Reaktion wird gestoppt durch kurzes Abspülen der Objektträger mit Aqua dest..

Auf die noch feuchten Ojektträger werden 1-2 Tropfen erhitzter Kaisers Gelatine gegeben und mit einem Deckglas eingedeckt.

## Ergebnis der Immunanfärbung von Mykobakterien:

Wenn Sputum nach der Verflüssigung der erfindungsgemäßen Wärme-Tensidbehandlung unterworfen wird und man es wie beschrieben enzym-immunologisch anfärbt, so sind Mykobakterien im Mikroskop als braun-rote Stäbchen erkennbar, die an den Polen eine wesentlich dunklere, kappenähnliche Färbung aufweisen. Verglichen mit der Ziehl-Neelson-Färbung erscheinen die Mykobakterien etwa doppelt so lang und wesentlich dicker. Besonders bei Sputum mit geringerem Mykobakteriengehalt wird die Untersuchung erleichtert. Werden die Sputen nicht vor der Färbung der erfindungsgemäßen Wärme-Tensidbehandlung unterworfen, so sind nach der Immunanfärbung Mykobakterien nicht oder nur äußerst unscharf zu erkennen.

## Beispiel 2

## Immunanfärbung von Mykobakterien im Sputum mit monoklonalen Antikörpern

Die Verflüssigung der Sputumprobe und die Herstellung der Präparate erfolgt wie in Beispiel 1, Stufen A. und B. beschrieben.

## C. Immunanfärbung mit monoklonalen Antikörpern:

Präparate gemäß Beispiel 1B. werden mit 200 µl Hybridomaüberstand des Klons BS104, 1:20 verdünnt in PBS-Polyoxyethylensorbitanmonolaurat-Puffer, bedeckt und 20 min bei Raumtemperatur in der feuchten Kammer inkubiert. Nach dreimaligem Waschen der Präparate wird zum Nachweis einer Bindung Kaninchen anti-Maus IgG Peroxidase-markiert (Fa.Dako P260), 1:100 verdünnt in PBS-Polyoxyethylensorbitanmonolaurat-Puffer, 200 µl pro Präparat 20 min in feuchter Kammer inkubiert. Die nachfolgenden Schritte entsprechen denen der polyklonalen Anfärbung in Beispiel 1.

Ergebnis der Immunanfärbung von Mykobakterien mit dem monoklonalen Antikörper BS 104:

Die Anfärbung der Mykobakterien durch den Nachweis der Bindung des monoklonalen Antikörpers BS 104 entspricht im Prinzip der, wie sie nach Bindung von polyklonalen Antikörpern erreicht werden kann.

## Beispiel 3

### Immunanfärbung von Mykobakterien im Sputum mit an Latexpartikel gebundenen monoklonalen Antikörpern

1 ml Carboxyllatex mit 2,5% Festgewicht wird 3 $\times$ in destilliertem Wasser gewaschen, abzentrifugiert und zum Schluß wieder in destilliertem Wasser resuspendiert. 0,25 ml 0,05 M Phosphatpuffer pH 4,5 werden zugegeben. Bei einer Temperatur von 20-25°C werden 1,25 ml einer 2%igen EDAC (1-Ethyl-3-(dimethylaminopropyl)-carbodiimid) Lösung zugegeben. Nach 4 Stunden wird mit 0,9% Kochsalzlösung gewaschen, zentrifugiert und in 1 ml 0,9%iger Kochsalzlösung resuspendiert. 1 ml dieser aktivierten Latexsuspension wird zusammengegeben mit

A) 0,3 mg einer F(ab)$_2$-Fraktion von BS 104 in 1 ml 0,2M Boratpuffer pH 8,5,
B) 0,3 mg BS 104 wie unter A)
C) 0,3 mg BS 100 wie unter A)
D) 0,3 mg BS 102 wie unter A)
E) 0,3 mg BS 107 wie unter A)
F) 0,3 mg F(ab)$_2$ Fraktion BS 107 wie unter A).

Danach wird noch 20 Stunden bei Raumtemperatur stehengelassen und anschließend 2,5 ml einer 5 millimolaren Lösung von Ethanolamin zugegeben. Dann wird 1 ml einer 2%igen Rinderserumalbuminlösung zugegeben. Nach weiteren 4 Stunden wird der Antikörperlatex in 0,9% Kochsalz gewaschen, anschließend in 0,1M Glycinpuffer pH 8,2 mit 0,05% Polyoxyethylensorbitanmonolaurat. Nach 4 maligem Waschen wird abzentrifugiert und in 0,1M Glycinpuffer pH 8,2 mit 0,9% NaCl, 0,1% NaN$_3$, 0,2% Rinderalbumin und 0,05% Polyoxyethylensorbitanmonolaurat aufgenommen und bei 4°C gelagert.

Nach Rezept 4 verflüssigtes Sputum mit M.tb. Bakterien wird zusammengegeben mit:

I) Carboxyllatex + A
II) Carboxyllatex + F
III) Carboxyllatex + D + E zu gleichen Teilen
IV) Carboxyllatex + C + D zu gleichen Teilen.

Der Latex wird über eine Mikrofiltrationsplatte mit 0,45 $\mu$ Filterboden abfiltriert.

Die Vertiefung mit I wird mit 0,05 mg/ml BS 102 + BS 100 versetzt
Die Vertiefung mit II wird mit 0,05 mg/ml BS 104 versetzt
Die Vertiefung mit III wird mit 0,05 mg/ml BS 104 Peroxidase markiert
Die Vertiefung mit IV wird mit 0,05 mg/ml BS 107 Peroxidase markiert

Nach 5 Minuten wird 10 mal mit Wasser mit 0,05 % Polyoxyethylensorbitanmonolaurat gewaschen. Die Vertiefungen mit I und II werden mit Protein A - Peroxidase (0,02 mg/ml) versetzt und nach 5 Minuten 10 mal gewaschen.

Danach wird mit Enzymsubstratfärbelösung gemäss Rezept 3 versetzt. Es tritt eine deutliche Rotfärbung auf.

Bei Verwendung von Tetramethylbenzidin anstelle von Aminoethylcarbazol tritt eine Blaufärbung auf.

## Beispiel 4

Der Versuch von Beispiel 3 wird wiederholt mit einer Aufschwemmung von M.tb. aus Kultur. Bis zu einer Keimzahl von 100-300 ml tritt noch eine deutliche Färbung ein. Wenn eine Aufschwemmung von Nocardien, Pseudomonaden oder E.coli eingesetzt wird, tritt keine Färbung auf.

## Beispiel 5

Charakterisierung der verwendeten monoklonalen BS-Antikörper.

Intakte Ganzzellen von Bakterien werden an Polystyrolmikrotiterplatten gebunden und 1:20 bis 1:500 verdünnte Kulturüberstände von BS 100, BS 102, BS 104 und BS 107 damit inkubiert.

Die gebundenen Antikörper werden mit Peroxidasemarkiertem anti Maus IgG nachgewiesen.

Im Western blot werden Proteinbanden von M.tb. mit den Antikörpern umgesetzt und mit anti Maus IgG Peroxidasemarkiert nachgewiesen. Die Ergebnisse sind aus der Tabelle ersichtlich.

Die die Antikörper BS 100, 102, 104 und 107 produzierenden Zell-Linien der gleichen Bezeichnung wurden bei der C.N.C.M.am 22. Dezember 1986 unter folgenden Nummern hinterlegt:

BS 100 = I-644
BS 102 = I-645
BS 104 = I-646
BS 107 = I-647.

Reaktion im Enzymimmunoassay mit:

| Antikörper | Molekulargewicht der erkannten Antigenbande im Western blot | $H_{37}R_v$ M. tb. | BCG | M. avium | M. kansasii | M. scro-fulaceum | M. fortui-tum | M. smeg-matis | M. phlei | M. chelo-nei | Pseud-omonas | Coryne-bakt. | E. coli |
|---|---|---|---|---|---|---|---|---|---|---|---|---|---|
| BS 100 | 60 – 65000 | 0,299 | 0,018 | 0,025 | 0,023 | 0,002 | 0,005 | 0,013 | 0,001 | 0,008 | 0,025 | 0,000 | 0,025 |
| BS 102 | 65000 | 1,459 | 0,023 | 0,024 | 0,031 | 0,005 | 0,017 | 0,003 | 0,000 | 0,000 | 0,013 | 0,002 | 0,005 |
| BS 104 | 71000 | 1,297 | 0,034 | 0,010 | 0,015 | 0,003 | 0,014 | 0,000 | 0,005 | 0,005 | 0,032 | 0,010 | 0,070 |
| BS 107 | Triplett 48000, 55000, 65000 | 2,000 | 0,000 | 0,040 | 0,052 | 0,000 | 0,079 | 0,009 | 0,000 | 0,000 | 0,003 | 0,000 | 0,008 |

## Ansprüche

1. Verfahren zum immunologischen Nachweis von Mykobakterien im Sputum, dadurch gekennzeichnet, daß man eine Sputumprobe einer an sich bekannten Vorbehandlung zur Dekontaminierung und Verflüssigung des Sputums unterwirft, die verflüssigte Probe zentrifugiert, den Überstand abgießt, das Sediment mit 2-3 Teilen je 1 Teil Sputum eines anionischen, zwitterionischen oder nichtionischen Tensids aus der Gruppe Natriumdodecylsulfat, zwitterionische Sulfobetaine, Glucopyranoside, Polyglykolether, Polyoxyethylenether und Polyoxyethylensorbitane versetzt, 2 - 4 Minuten im Wasserbad bei 80 bis 100°C erhitzt, ggf. grobe Schleimflocken abzentrifugiert, die verflüssigte, so behandelte Sputumprobe ausstreicht, den Ausstrich trocknet, fixiert, mit einem geeigneten Puffer mit oder ohne Tensid rehydriert, ein oder mehrmals in einem Pufferbad wäscht, den Ausstrich wiederum sorgfältig trocknet, mit einem markierten polyklonalen oder monoklonalen Antikörper gegen Mykobakterien in Gegenwart von Feuchtigkeit bei Zimmertemperatur bis 37°C inkubiert, wobei der Ausstrich mit Antikörper vollständig bedeckt sein und feucht gehalten werden muß, ein oder mehrmals mit einem geeigneten Puffer wäscht und die Probe einer fur die Markierung geeigneten immunologischen Nachweismethode unterwirft.

2. Weitere Ausgestaltung des Verfahrens gemäß Anspruch 1, dadurch gekennzeichnet, daß man den Ausstrich zunächst mit einem nicht markierten Antikörper bei Zimmertemperatur inkubiert, ein oder mehrmals mit einem geeigneten Puffer mit oder ohne Tensid wäscht und anschließend mit einem markierten zweiten Antikörper inkubiert und dann weiter nach Anspruch 1 verfährt.

3. Verfahren nach Anspruch 1 oder 2, dadurch gekennzeichnet, daß man monoklonale Antikörper mit Spezifität gegen M. tuberculosis zum Nachweis von Tb oder mit Spezifität gegen M. avium, scrofulaceum, kansasii, smegmatis, phlei, fortuitum oder chelonei zum Nachweis von ubiquitär vorkommenden (atypischen) Mykobakterien verwendet.

4. Verfahren nach einem der vorstehenden Ansprüche, dadurch gekennzeichnet, daß man monoklonale Antikörper verwendet, die von den Hybridoma-Zell-Linien BS 100 (CNCM I-644 ), BS 102 (CNCM I-645 ), BS 104 (CNCM I-646 ) und BS 107 (CNCM I-647 ) produziert worden sind.

5. Verfahren nach einem der vorstehenden Ansprüche, dadurch gekennzeichnet, daß man an Latexpartikel gebundene monoklonale Antikörper verwendet.

6. Verfahren nach einem der vorstehenden Ansprüche, dadurch gekennzeichnet, daß man den Antikörper mit einem Enzym markiert und die Enzymmarkierung mit einem geeigneten Farbsubstrat sichtbar macht.

7. Verfahren nach Anspruch 6, dadurch gekennzeichnet, daß man als Enzym Peroxidase verwendet und eventuell vorhandene Gewebsperoxidase im Ausstrich nach der Rehydrierung und vor der Inkubation mit Antikörper inaktiviert.

8. Hybridoma-Zell-Linien BS 100 (CNCM I-644 ), BS 102 (CNCM I-645), BS 104 (CNCM I-646 ) und BS 107 (CNCM I-647).

9. Monoklonale Antikörper, die von den Hybridoma-Zell-Linien gemäß Anspruch 8 produziert worden sind.